# EUROPEAN PATENT SPECIFICATION

(11) **EP 0 553 874 B1**
(45) Date of publication and mention of the grant of the patent: **21.08.1996**
(21) Application number: 93101444.3
(22) Date of filing: 29.01.1993
(51) Int. Cl.: C07C 37/08, C07C 39/07

(54) **Process for production of cresols**
Verfahren zur Herstellung von Kresolen
Procédé pour la préparation de crésols

(30) Priority: 31.01.1992 JP 16136/92
(43) Date of publication of application: 04.08.1993
(73) Proprietor: SUMITOMO CHEMICAL COMPANY, LIMITED, Osaka-shi, Osaka (JP)
(72) Inventor: Ikimi, Kiyoshi, Oita-shi, Oita-ken (JP); Ikeda, Yoichi, Oita-shi, Oita-ken (JP); Murakami, Akira, Oita-shi, Oita-ken (JP); Okamoto, Kazushige, Oita-shi, Oita-ken (JP); Tokumaru, Tooru, Oita-shi, Oita-ken (JP); Hazama, Motoo, Oita-shi, Oita-ken (JP)
(74) Representative: VOSSIUS & PARTNER

(56) References cited:
- EP-A- 0 077 749
- DE-A- 2 650 416
- CHEMICAL ABSTRACTS, vol. 88, no. 25, 19 June 1978, Columbus, Ohio, US; abstract no. 190380, page 722 ;column 1 ;
- CHEMICAL ABSTRACTS, vol. 100, no. 21, 21 May 1984, Columbus, Ohio, US; abstract no. 174433, page 595 ;column 2 ;

## Description

The present invention relates to a process for the production of cresols.

As a process for cresol production, there has been widely known a process in which cymene is oxygenated with oxygen gas and tertiary hydroperoxide of cymene is then decomposed into the desired cresols and acetone.

In this process, however, a primary hydroperoxide of cymene with an oxygenated methyl group is formed as a by-product in the oxygenation, together with the above tertiary hydroperoxide. The primary hydroperoxide is converted into isopropylphenol and formaldehyde through its decomposition. This formaldehyde may be condensed with the resulting cresols to form a resin, which will cause a decrease in the yield of cresols in this process.

To solve this problem, a process has been proposed, in which the decomposition of the primary hydroperoxide is stopped halfway to suppress the formation of formaldehyde, so that a yield decrease arising from the by-product can be prevented, and the remaining primary hydroperoxide is then hydrogenated into an alkylbenzene (see, e.g., JP-A 52-57130, JP-B 59-8246, JP-B 1-49248).

In this process, however, a portion of the primary hydroperoxide still decomposes. Therefore there will inevitably occur to a certain degree the side reaction between formaldehyde as the by-product and cresols as the major product, so that a loss of cresol yield will accompany the process. This process is not satisfactory with respect to the yield of cresols.

For the purpose of preventing the formation of formaldehyde, a process has been known, in which a mixture of the tertiary and primary hydroperoxides obtained by the oxygenation is subjected to hydrogenation below 50°C to decrease the content of primary hydroperoxide (see, e.g., JP-A 58-198468). In this process, a yield decrease arising from the formation of formaldehyde can be suppressed. However, the yield of cresols in this process cannot be satisfactory either.

Object of the present invention is to prevent the disadvantages of the prior art above.
As a solution, the present invention has been completed with the novel finding that cresols can be obtained in high yield from consumed cymene because a yield decrease arising from the formation of formaldehyde can be prevented and the primary hydroperoxide and other by-products also can be selectively recovered as the raw material cymene. The process is conducted by subjecting a solution of oxygenation products containing the tertiary and primary hydroperoxides of cymene, which has been obtained by oxygenation of cymene with oxygen gas or an oxygen-containing gas, to hydrogenation in advance of decomposition for the purpose of decreasing the content of primary hydroperoxide, subjecting the solution to decomposition and then hydrogenation again.

That is, the present invention provides a process for the production of cresols, comprising the steps of: (a) conducting oxygenation of cymene with oxygen gas or an oxygen-containing gas to obtain a solution of oxygenation products containing tertiary and primary hydroperoxides of cymene; (b) subjecting the solution of oxygenation products obtained in the step (a) to hydrogenation to decrease the content of primary hydroperoxide; (c) subjecting the solution treated in the step (b) to decomposition in the presence of a catalyst; and (d) subjecting the solution treated in the step (c) to hydrogenation.

The following will describe the step of conducting the oxygenation of cymene with oxygen gas or an oxygen-containing gas to obtain a solution of oxygenation products containing tertiary and primary hydroperoxides of cymene.

Examples of the raw material cymene which can be used in this step are in various forms such as o-cymene, m-cymene and p-cymene. Among these cymene isomers, any single isomer can be used solely or mixtures thereof in any ratio can also be used.

This step can be performed by ordinary oxygenation in liquid phase, and usually attained by bringing cymene in contact with oxygen gas or an oxygen-containing gas such as air.

The oxygenation is usually conducted under normal pressure or under pressure. The pressure of oxygen gas or an oxygen-containing gas is usually in the range of from 0-1,96 MPa (0 to 20 kg/cm²) as a gauge pressure.

In this step, an alkali may be allowed to coexist in the reaction system, examples of which are carbonates of alkali metals, such as sodium carbonate and potassium carbonate; hydroxides of alkaline earth metals, such as magnesium hydroxide and calcium hydroxide; carbonates of alkaline earth metals, such as calcium carbonate; amines such as pyridine, piperidine and triethylamine; and ammonia.

In this step, an initiator may be added to the reaction system, examples of which are azo compounds such as 2,2'-azobisisobutyronitrile, and peroxides such as benzoyl peroxide and cymene hydroperoxide. The amount of initiator to be used is usually in the range of from 0.01% to 5% by weight, based on the weight of cymene.

The reaction temperature is usually in the range of from 30° to 200°C, preferably 80° to 150°C. After completion of the reaction, a solution of oxygenation products is obtained, which may be further subjected, if necessary, to an ordinary post-treatment such as fractionation with a separatory funnel or filtration. This solution of oxygenation products can be used in the subsequent step without undergoing a particular post-treatment; in case where no alkali is used in this step, however, the resulting solution of oxygenation products may be washed, before its use in the subsequent step, with an aqueous solution of alkali substances such as hydroxides of alkali metals, carbonates of alkali metals, hydroxides of alkaline earth metals or carbonates of alkaline earth metals.

The following will describe the step of subjecting the solution of oxygenation products obtained in the fore-going step to hydrogenation to decrease the content of the primary hydroperoxide.

This step can be performed by ordinary catalytic hydrogenation, and usually attained by introducing hydrogen gas under normal pressure or under pressure in the presence of a catalyst. The pressure of hydrogen gas is usually in the range of from 0-1,96 MPa (0 to 20 kg/cm²) as a gauge pressure.

Examples of the catalyst which can be used in this step are those composed of a metal such as Pd, Pt, Ni, Ru, Rh or Re. These catalysts can also be used in a supported form on a carrier such as active carbon, titania, zirconia, silica-magnesia, alumina or alumina-magnesia. Preferred are Pd catalysts and Ni catalysts. More preferred are Pd/C, Pd/alumina, Pd/TiO₂, Pd/alumina-magnesia, Pd/silica-magnesia and Raney Ni. The amount of catalyst to be used is usually in the range of from 0.001% to 1% by weight, based on the weight of the solution of oxygenation products.

The reaction temperature is usually in the range of from 10° to 120°C, preferably 20° to 100°C, more preferably 50° to 100°C.

By this hydrogenation, the primary hydroperoxide is converted into a compound which will give no more formaldehyde in the subsequent decomposition step, and hence there is no possibility that the yield of cresols may be decreased by the side reaction in the decomposition step. Moreover, by the additional hydrogenation after the decomposition, the compounds produced in the first hydrogenation step of the primary hydroperoxide, as well as the other by-products in the reaction mixture resulted in the decomposition, are converted into the raw material cymene which will be recycled. Thus, the yield of cresols from consumed cymene will finally increase.

In the hydrogenation, the tertiary hydroperoxide can also be reacted, together with the primary hydroperoxide. The reaction rate of the tertiary hydroperoxide is considerably lower than that of the primary hydroperoxide, and the reaction products obtained from the tertiary hydroperoxide by the hydrogenation can be recycled as the raw material, if any; therefore, the hydrogenated tertiary hydroperoxide will not affect the yield of cresols from consumed cymene. It is, however, disadvantageous from an economical point of view that the hydrogenation is conducted to a degree than required. It is usually advantageous from an economical point of view, i.e., the yield of cresols through one pass and the energy efficiency in the process, that the hydrogenation is conducted in such a manner that the weight ratio of primary hydroperoxide to tertiary hydroperoxide is usually decreased to not greater than 1/25 (w/w), preferably not greater than 1/50 (w/w), more preferably not greater than 1/100 (w/w), and that the degree of hydrogenation of tertiary hydroperoxide as the precursor of cresols is 30% or less, preferably 20% or less, more preferably 15% or less. These results of hydrogenation can be attained by adjusting the reaction temperature and pressure for hydrogenation, as well as the kind and amount of catalyst to be used for hydrogenation, to the above prescribed conditions.

In this step, the solution thus treated may be subjected to an analysis such as liquid chromatography to check the degrees of conversion of primary and tertiary hydroperoxides, which also makes it possible to determine the end point of the reaction.

After completion of the reaction, the catalyst is removed, for example, by filtration, and the residue may be used in the subsequent step, if necessary, after subjected to a post-treatment such as alkali washing and concentration.

The following will describe the step of subjecting the solution obtained in the foregoing step to decomposition in the presence of a catalyst.

Examples of the catalyst which can be used in this step are acidic catalysts, sulfur and metal complex catalysts such as Burmah catalyst. Specific examples of the acidic catalyst are inorganic acids such as sulfuric acid, hydrochloric acid, perchloric acid, SO₂ and SO₃; organic acids such as benzenesulfonic acid, p-toluenesulfonic acid, cresolsulfonic acid and chloroacetic acid; solid acids such as silica-alumina, alumina and strongly acidic ion exchange resin; heteropolyacids such as tungstosilicic acid, tungstophosphoric acid and molybdophosphoric acid. The Burmah catalyst refers to a catalyst of the general formula: 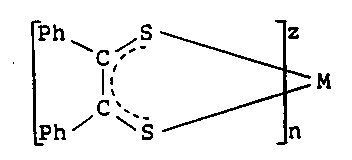 wherein M is Ni, Pd or Fe(II); Ph is a phenyl group which may be optionally substituted with at least one substituent; n is an integer of 1, 2 or 3; z is a formal charge of the complex, selected from 0, -1 and -2. Examples of the Burmah catalyst are bis(dithiobenzil)nickel, bis(dithiobenzil)palladium and bis(dithiobenzil)iron(II). Preferred are sulfuric acid and cresolsulfonic acid. The amount of catalyst to be used, although it can be determined depending upon the kind of that catalyst, is usually in the range of from about 0.0001% to 1% by weight, based on the weight of the solution obtained after the hydrogenation.

The reaction temperature is usually in the range of from 30° to 150°C.

In this step, the solution thus treated may be further subjected to an analysis such as liquid chromatography to check the degree of decomposition of hydroperoxides.

By this decomposition, the tertiary hydroperoxide is decomposed into the desired cresols and acetone.

After completion of the reaction, the reaction mixture can be used in the subsequent step without undergoing a particular post-treatment, or if necessary, after subjected to a post-treatment such as filtration and neutralization. The reaction mixture can also be used in the subsequent step after the removal of produced acetone.

The following will describe the step of subjecting the solution thus treated to hydrogenation.

This step can be performed by ordinary catalytic hydrogenation, and usually attained by introducing hydrogen gas in the reaction system under normal pressure or under pressure in the presence of a catalyst. The pressure of hydrogen gas is usually in the range of from 0-9,8 MPa (0 to 100 kg/cm²) as a gauge pressure.

Examples of the catalyst which can be used in this step are those composed of a metal such as Pd, Cr, Cu, Pt, Ni, Ru, Rh or Re. These catalysts can also be used in a supported form on a carrier such as active carbon, titania, zirconia, silica-magnesia, alumina, alumina-magnesia or strongly acidic ion exchange resin. Preferred are those composed of Pd or Cu-Cr. More preferred are Pd/C, Pd/alumina, Pd/TiO₂, Cu-Cr/C, Cu-Cr/TiO₂ and Pd/strongly acidic ion exchange resin. The amount of catalyst to be used is usually in the range of from 0.001% to 20% by weight, based on the weight of the solution after the decomposition.

In this step, any other catalyst may be allowed to coexist in the reaction system, if necessary. Examples of such a catalyst are the same catalysts as used in the decomposition step. The reaction mixture after the decomposition may be used as it is, without undergoing the removal of the catalyst used in the decomposition step.

The reaction temperature is usually in the range of from 0° to 250°C, preferably 20° to 250°C.

After completion of the reaction, the removal of the catalyst by filtration gives cresols, cymene and acetone, which can be separated and purified, if necessary, by neutralization and then distillation. The portion of cymene obtained by the hydrogenation can be recovered, together with the unreacted portion of cymene, and both can be recycled as the raw material in the process of the present invention.

The process of the present invention can be performed either by a batch method or a continuous method.

The process of the present invention can afford cresols in high yield because not only the primary hydroperoxide but also other by-products are recovered as cymene by two hydrogenation steps, i.e., one after the oxygenation of cymene with oxygen gas or an oxygen-containing gas and the other after the decomposition reaction of tertiary hydroperoxides into cresols.

Accordingly, the formation of formaldehyde from the primary hydroperoxide, hence a yield decrease thereby, is almost negligible.

The process of the present invention has an additional advantage that the resulting cresols can readily be isolated from the by-products.

The present invention will be illustrated by way of the following examples which are not to be construed to limit the scope thereof.

The abbreviations used in the Examples are shown below together with their corresponding chemical structures.

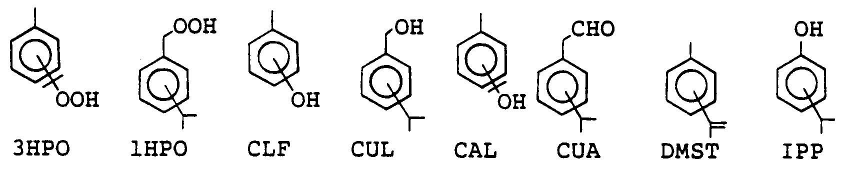

### Example 1

I. In a reaction vessel equipped with a stirrer, an air-blowing tube, a thermometer and a condenser, placed are cymene (807.0 g; content, 99.3%) and a cymene solution (78.0g; cymene content, 82.1%; 3HPO content, 11.2%; and 1HPO content, 0.65%) containing cymene hydroperoxide. The reaction is conducted, while blowing air thereinto, at 120°C under normal pressure for 5 hours. The reaction mixture is then washed with 1% aqueous sodium hydroxide (90 g) to give 909.2 g of a hydroperoxide mixture-containing oil phase (composition: cymene, 81.9%; 3HPO, 12.1%; 1HPO, 2.2%; CAL, 1.0%; CUL, 0.23%; CUA, 0.20%; CLF, 0.01%; and others, 2.4%).
II. The hydroperoxide mixture-containing oil phase (909.2 g) obtained in Sec. I. of this Example and 1% palladium-titania catalyst (0.45 g) are placed in a flask. Hydrogenation is conducted with stirring, while blowing hydrogen gas thereinto, at 70°C under normal pressure for 1 hour. After completion of the reaction, the removal of the catalyst by filtration gives 909.2 g of the reaction mixture (composition: cymene, 81.8%; 3HPO, 11.0%; 1HPO, 0.03%; CAL, 2.4%; CUL, 1.7%; CUA, 0.72%; CLF, 0.01%; and others, 2.2%). The weight ratio of 1HPO to 3HPO in the reaction mixture is 1/370, and the degree of conversion is 98.7% for 1HPO and 8.7% for 3HPO, respectively.
   From this reaction mixture, the unreacted portion (699.8 g) of cymene is removed by distillation at 70°C under a pressure of from 1,33 to 2,66 kPa (10 to 20 mmHg), 209.3 g of the concentrate is obtained (composition: cymene, 21.0%; 3HPO, 47.8%; 1HPO, 0.13%; CAL, 10.5%; CUL, 7.6%; CUA, 3.1%; CLF, 0.04%; and others, 9.8%).
III. Concentrated sulfuric acid (0.17 g) and acetone (29 g) are placed in a flask, and the mixture is heated to reflux, to which the concentrate (209.3 g) obtained in Sec. II. of this Example is added dropwise under reflux. The reaction mixture is maintained at 65°C for 0.5 hours. After completion of the reaction, 238.3 g of the solution of decomposition products is obtained (composition: cymene, 18.3%; 3HPO, not found; 1HPO, not found; CAL, 0.88%; CUL, 5.6%; CUA, 2.6%; DMST, 5.1%; CLF, 26.0%; acetone, 26.9%; and others, 14.7%). The yield of cresol from consumed cymene is 59.4%.
IV. In an autoclave made of stainless steel, placed are the solution of decomposition products (238.3 g) obtained in Sec. III. of this Example and 1% palladium-titania catalyst (4.7 g). The reaction is conducted, while introducing hydrogen gas thereinto under a hydrogen gauge pressure of 392,2 kPa (4 kg/cm²), at 75°C for 2 hours. After completion of the reaction, the catalyst is removed by filtration, and the resulting solution is neutralized by addition of aqueous sodium hydroxide until the water layer has pH 7. From the organic layer obtained, acetone is removed by distillation under normal pressure, and the residue is then distilled under reduced pressure to give 63.0 g of cresol and 82.1 g of cymene. The yield of cresol from consumed cymene is 86%.

### Example 2

I. In a reaction vessel equipped with a stirrer, an air-blowing tube, a thermometer and a condenser, placed are cymene (820.3 g; content, 99.9%) and a cymene solution (65.4 g; cymene content, 84.06%; 3HPO content, 8.51%; and 1HPO content, 0.47%) containing cymene hydroperoxide. The reaction is conducted, while blowing air thereinto, at 120°C under normal pressure for 5 hours. After completion of the reaction, 897.2 g of a hydroperoxide mixture-containing oil phase (composition: cymene, 90.0%; 3HPO, 7.6%; 1HPO, 1.3%; CAL, 0.45%; CUL, 0.07%; CUA, 0.09%; CLF, <0.01%; and others, 0.44%) is obtained.
II. The hydroperoxide mixture-containing oil phase (897.2 g) obtained in Sec. I. of this Example and 1% palladium-carbon catalyst (0.45 g) are placed in a flask. Hydrogenation is conducted with stirring, while blowing hydrogen gas thereinto, at 27°C under normal pressure for 1 hour. After completion of the reaction, the catalyst is removed by filtration, and the reaction mixture is washed with 1% aqueous sodium carbonate (90 g) to give 896.0 g of the reaction mixture (composition: cymene, 90.1%; 3HPO, 6.7%; 1HPO, 0.13%; CAL, 1.4%; CUL, 0.89%; CUA, 0.53%; CLF, 0.01%; and others, 0.18%). The weight ratio of 1HPO to 3HPO in the reaction mixture is 1/52, and the degree of conversion is 90.0% for 1HPO and 11.7% for 3HPO, respectively.
   From this reaction mixture, the unreacted portion (775.1 g; purity, 99.7%) of cymene is removed by distillation at 70°C under a pressure of 2,66 kPa (20 mmHg) to give 115.2 g of the concentrate (composition: cymene, 24.8%; 3HPO, 51.7%; 1HPO, 0.96%; CAL, 10.3%; CUL, 6.8%; CUA, 3.9%; CLF, 0.07%; and others, 1.5%).
III. Concentrated sulfuric acid (0.092 g) and acetone (14 g) are placed in a flask, and the mixture is heated to reflux, to which the concentrate (115.2 g) obtained in Sec. II. of this Example is added dropwise under reflux. The reaction mixture is maintained at 65°C for 0.5 hours. After completion of the reaction, 129.2 g of the solution of decomposition products is obtained (composition: cymene, 22.4%; 3HPO, not found; 1HPO, not found; CAL, 0.57%; CUL, 5.1%; CUA, 3.4%; DMST, 5.6%; CLF, 29.9%; acetone, 27.0%; and others, 6.1%). The yield of cresol from consumed cymene is 60.7%.
IV. In an autoclave made of stainless steel, placed are the solution of decomposition products (129.2 g) obtained in Sec. III. of this Example and 5% palladium-carbon catalyst (1.29 g). The reaction is conducted, while introducing hydrogen gas thereinto under a hydrogen gauge pressure of 294,2 kPa (3 kg/cm²), at 100°C for 2.5 hours. After completion of the reaction, the catalyst is removed by filtration, and the resulting solution is neutralized by addition of aqueous sodium hydroxide until the water layer has pH 7. From the organic layer obtained, acetone is removed by distillation under normal pressure, and the residue is then distilled under reduced pressure to give 31.5 g of cresol and 46.1 g of cymene. The yield of cresol from consumed cymene is 82.2%.

### Comparative Example 1

I. In a reaction vessel equipped with a stirrer, an air-blowing tube, a thermometer and a condenser, placed are cymene (813.1 g; content, 99.0%) and a cymene solution (72.2 g; cymene content, 82.0%; 3HPO content, 12.8%; and 1HPO content, 0.44%) containing cymene hydroperoxide. The reaction is conducted, while blowing air thereinto, at 120°C under normal pressure for 5 hours. The reaction mixture is then washed with 1% aqueous sodium hydroxide (90 g) to give 905.5 g of a hydroperoxide mixture-containing oil phase (composition: cymene, 82.6%; 3HPO, 11.1%; 1HPO, 2.1%; CAL, 1.1%; CUL, 0.23%; CUA, 0.21%; CLF, 0.03%; and others, 2.6%).
   From this reaction mixture, the unreacted portion (687.2 g) of cymene is removed by distillation at 60°C under a pressure of from 1,33 - 4 kPa (10 to 30 mmHg) to give 210.7 g of the concentrate (composition: cymene, 31.5%; 3HPO, 47.4%; 1HPO, 9.2%; CAL, 4.6%; CUL, 1.1%; CUA, 0.85%; CLF, 0.07%; others, 5.4%)
II. Concentrated sulfuric acid (0.19 g) and acetone (27 g) are placed in a flask, and the mixture is heated to reflux, to which the concentrate (210.7 g) obtained in Sec. I. of this Example is added dropwise under reflux. The reaction mixture is maintained at 65°C for 0.5 hours. After completion of the reaction, 237.9 g of the solution of decomposition products is obtained (composition: cymene, 28.3%; 3HPO, not found; 1HPO, 1.20%; CAL, 0.15%; CUL, 1.6%; CUA, 1.3%; DMST, 1.6%; CLF, 26.3%; acetone, 26.1%; and others, 13.4%). The degree of conversion is 100% for 3HPO and 85.3% for 1HPO, respectively. The yield of cresol from consumed cymene is 63.5%.
III. In an autoclave made of stainless steel, placed are the solution of decomposition products (237.9 g) obtained in Sec. II. of this Example and 1% palladium-titania catalyst (4.76 g). The reaction is conducted, while introducing hydrogen gas thereinto under a hydrogen gauge pressure of 490 kPa (5 kg/cm²), at 75°C for 2 hours. After completion of the reaction, the catalyst is removed by filtration, and the resulting solution is neutralized by addition of aqueous sodium hydroxide until the water layer has pH 7. From the organic layer obtained, acetone is removed by distillation under normal pressure, and the residue is then distilled under reduced pressure to give 61.6 g of cresol and 84.7 g of cymene. The yield of cresol from consumed cymene is 72.8%.

### Comparative Example 2

An acetone solution (0.6 ml) having an adjusted sulfuric acid concentration of 3.29 × 10⁻³ g/ml is placed, together with acetone (4.4 ml), in a flask. The mixture is heated to reflux, to which the concentrate (30.0 g; composition: cymene, 31.5%; 3HPO, 47.4%; 1HPO, 9.2%; CAL, 4.6%; CUL, 1.1%; CUA, 0.85%; CLF, 0.07%; and others, 5.4%) obtained in the same manner as described in Sec. I of Comparative Example 1 is added dropwise under reflux. The reaction mixture is maintained at 65°C for 0.5 hours. After completion of the reaction, 33.9 g of the solution of decomposition products is obtained (composition: cymene, 28.1%; 3HPO, 32.5%; 1HPO, 5.8%; CAL, 3.2%; CUL, 1.3%; CUA, 0.69%; DMST, 1.7%; CLF, 3.4%; acetone, 15.4%; and others, 7.9%). The degree of conversion is 22.5% for 3HPO and 28.7% for 1HPO, respectively.

### Examples 3 to 9

Partial hydrogenation of a hydroperoxide mixture is conducted in the same manner as described in Sec. II. of Example 1, except for the conditions shown in Table 1. The composition of the hydroperoxide mixture used is as follows: cymene, 81.9%; 3HPO, 12.1%; 1HPO, 2.2%; CAL, 1.0%; CUL, 0.23%; CUA, 0.20%; CLF, 0.01%; and others, 2.4%.

The results are shown in Table 1.

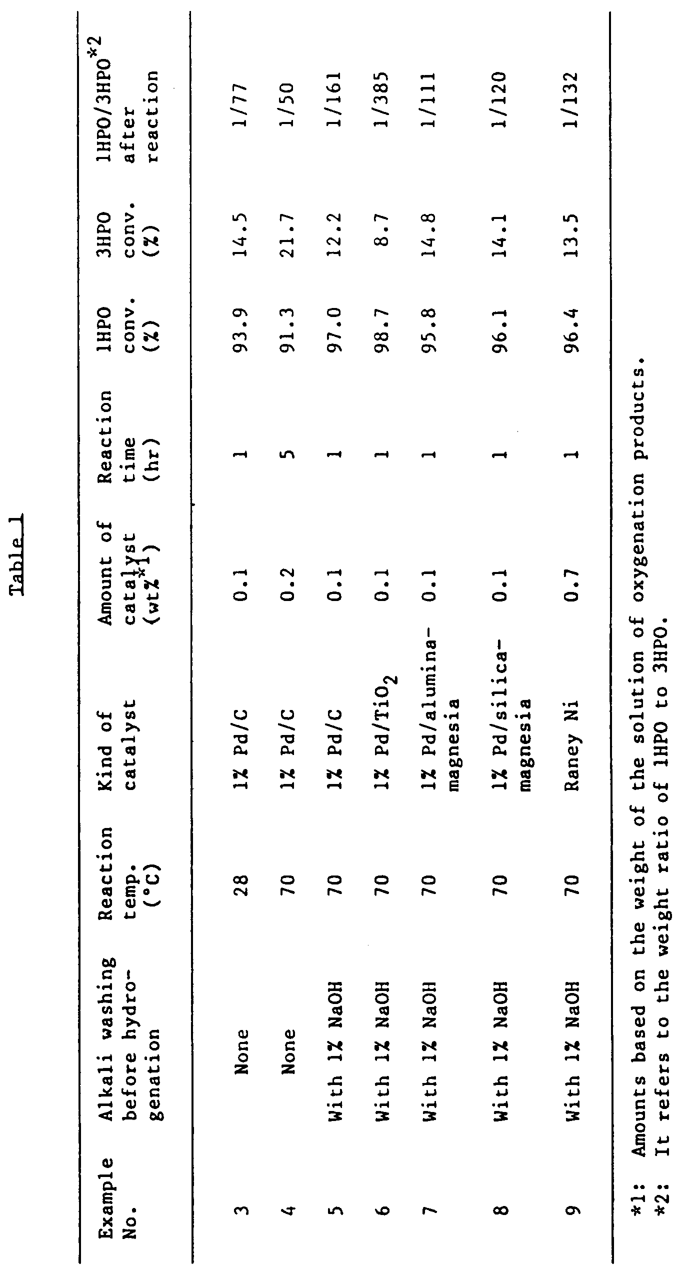

Each of the reaction mixture obtained in these Examples is concentrated and then subjected to the same operations as described in Secs.III. to IV. of Example 1, and cresols are thus obtained in high yield.

## Claims

1. A process for the production of cresols, comprising the steps of:
(a) conducting oxygenation of cymene with oxygen gas or an oxygen-containing gas, thereby obtaining a solution of oxygenation products containing tertiary and primary hydroperoxides of cymene;
(b) subjecting the solution of oxygenation products obtained in the step (a) to hydrogenation to decrease the content of primary hydroperoxide;
(c) subjecting the solution treated in the step (b) to decomposition in the presence of a catalyst; characterized in that the process further comprises
(d) subjecting the solution treated in the step (c) to hydrogenation to obtain cresols.

2. The process according to claim 1, wherein the hydrogenation in the step (b) is conducted in such a manner that the weight ratio of primary hydroperoxide to tertiary hydroperoxide is decreased to not greater than 1/25 (w/w).

3. The process according to claim 1 or 2, wherein the degree of hydrogenation of tertiary hydroperoxide is 30% or less.

4. The process according to anyone of claims 1 to 3, wherein the hydrogenation in the step (b) is conducted with a Pd catalyst or a Ni catalyst.

5. The process according to anyone of claims 1 to 4, wherein the hydrogenation in the step (d) is conducted with a Pd catalyst or a Cu-Cr catalyst.

## Patentansprüche

1. Verfahren zur Herstellung von Kresolen, umfassend die Schritte:
(a) Oxidieren von Cymol mit Sauerstoffgas oder einem sauerstoffhaltigen Gas, wodurch eine Lösung der Oxidationsprodukte erhalten wird, welche tertiäre und primäre Cymolhydroperoxide enthält,
(b) Hydrieren der in Schritt (a) erhaltenen Lösung der Oxidationsprodukte, um den Gehalt an primärem Hydroperoxid zu senken,
(c) Zersetzen der in Schritt (b) behandelten Lösung in Gegenwart eines Katalysators, dadurch gekennzeichnet, daß das Verfahren ferner
(d) Hydrieren der in Schritt (c) behandelten Lösung, wodurch Kresole erhalten werden,
umfaßt.

2. Verfahren nach Anspruch 1, wobei die Hydrierung in Schritt (b) so durchgeführt wird, daß das Gewichtsverhältnis des primären Hydroperoxids zum tertiären Hydroperoxid aufweniger als 1/25 (Gew./Gew.) abgesenkt wird.

3. Verfahren nach Anspruch 1 oder 2, wobei der Hydrierungsgrad des tertiären Hydroperoxids 30% oder weniger beträgt.

4. Verfahren nach einem der Ansprüche 1 bis 3, wobei die Hydrierung in Schritt (b) mit einem Pd-Katalysator oder einem Ni-Katalysator durchgeführt wird.

5. Verfahren nach einem der Ansprüche 1 bis 4, wobei die Hydrierung in Schritt (d) mit einem Pd-Katalysator oder einem Cu-Cr-Katalysator durchgeführt wird.

## Revendications

1. Procédé pour la production de crésols, comprenant les étapes suivantes :
(a) on effectue l'oxygénation du cymène par le gaz d'oxygène ou un gaz contenant de l'oxygène, en obtenant ainsi une solution de produits d'oxygénation contenant des hydroperoxydes de cymène teriaire et primaire ;
(b) on soumet à l'hydrogénation la solution de produits d'oxygénation obtenue dans l'étape (a) pour diminuer la teneur en hydroperoxyde primaire ; et
(c) on soumet la solution traitée dans l'étape (b) à la décomposition en présence d'un catalyseur, caractérisé en ce que le procédé comprend en outre l'étape suivante
(d) on soumet à l'hydrogénation la solution traitée dans l'étape (c) pour obtenir des crésols.

2. Procédé selon la revendication 1, dans lequel l'hydrogénation dans l'étape (b) est effectuée de telle manière que le rapport pondéral de l'hydroperoxyde primaire à l'hydroperoxyde tertiaire soit abaissé à pas plus de 1/25 en poids.

3. Procédé selon la revendication 1 ou 2, dans lequel le degré d'hydrogénation de l'hydroperoxyde tertiaire est de 30 % ou moins.

4. Procédé selon l'une quelconque des revendications 1 à 3, dans lequel l'hydrogénation dans l'étape (b) est effectuée avec un catalyseur au Pd ou un catalyseur au Ni.

5. Procédé selon l'une quelconque des revendications 1 à 4 dans lequel l'hydrogénation dans l'étape (d) est effectuée avec un catalyseur au Pd ou un catalyseur au Cu-Cr.
